# EUROPEAN PATENT APPLICATION

(11) **EP 4 176 936 A1**
(43) Date of publication of application: **10.05.2023**
(21) Application number: 22206032.9
(22) Date of filing: 08.11.2022
(51) Int. Cl.: A61Q 17/04, A61K 8/29, A61K 8/35, A61K 8/37, A61K 8/40, A61K 8/49, A61K 8/9789

(54) **COSMETIC COMPOSITION FOR PROTECTING THE SKIN AND HAIR AGAINST SOLAR RADIATION**

(30) Priority: 08.11.2021 EP 21383012
(71) Applicant: Martiderm, SL, 08758 Cervelló (ES)
(72) Inventor: VILASPASA TORRES, David, 08758 Cervelló (ES); CRESPO MOYA, Nuria, 08758 Cervelló (ES); SANCHO VILLALBA, Irene, 08758 Cervelló (ES); SUÑER OLLÉ, Elisa, 08758 Cervelló (ES)
(74) Representative: Torner, Juncosa I Associats, SL

(57) **Abstract**

The present invention discloses a use of a composition comprising a UV filter combination of Disodium 2,2'-(1,4-phenylene)bis(6-sulfo-1H-1,3-benzimidazole-4-sulfonate) (INCI: Disodium Phenyl Dibenzimidazole Tetrasulfonate), 2,2'-[6-(4-Methoxyphenyl)-1,3,5-triazine-2,4-diyl]bis{5-[(2-ethylhexyl)oxy]phenol} (INCI: Bis-Ethylhexyloxyphenol Methoxyphenyl Triazine), 4-[[4,6-bis[[4-(2-ethylhexoxy-oxomethyl)phenyl]amino]-1,3,5-triazin-2-yl]amino]benzoic acid 2-ethylhexyl ester (INCI: Ethylhexyl Triazone) and at least three compounds selected from the group of the six compounds consisting of 2-ethylhexyl 2-hydroxybenzoate (INCI: Ethylhexyl Salicylate), 3-(4-tert-Butylphenyl)-1-(4-methoxyphenyl)propane-1,3-dione (INCI: Butyl Methoxydibenzoylmethane), Titanium Dioxide, 3,3,5-Trimethylcyclohexyl 2-hydroxybenzoate (INCI: Homosalate), 2-Ethylhexyl 2-cyano-3,3-diphenylprop-2-enoate (INCI: Octocrylene) and 3-Methylbutyl (2E)-3-(4-methoxyphenyl)prop-2-enoate (INCI: Isoamyl p-Methoxycinammate) to increase the absorbance of a cosmetic preparation. The present invention also discloses a cosmetic preparation comprising the mentioned UV filters and a method to increase the absorbance of a cosmetic preparation comprising the mentioned composition including the step of irradiating the cosmetic preparation with UV light.

## Description

### TECHNICAL FIELD

The present disclosure relates to a cosmetic preparation, in particular, to a cosmetic preparation intended for protecting the skin and/or hair against solar radiation. The present disclosure also relates to a use of a composition as a cosmetic preparation.

### BACKGROUND ART

The trend away from a posh paleness towards a "healthy, sporty brown skin" is an undisputed tendency. In order to achieve this, people expose their skin to solar radiation, since it causes the formation of pigmentation in the form of melanin.

However, the ultraviolet radiation of solar radiation also has a damaging effect on the skin. Besides the acute damage (sunburn), also longer lasting damages such as an increased risk of skin cancer in case of excessive irradiation by light in the UVB range (wavelengths: 280-320 nm) occur. The excessive action of UVB and UVA radiation (wavelengths: 320-400 nm) also causes a weakening of the elastic and collagen fibers of the connective tissue. This in turn causes several phototoxic and photoallergic reactions and a premature skin aging.

In order to protect the skin, a series of light protection filtering substances has been developed, which may be used in cosmetic preparations. These UVA and UVB filters generally are summarized in most industrial countries in positive lists such as in Annex 7 of the Cosmetic Regulation.

In 2006 the European Commission published recommendation 2006/647/EC regarding the efficacy of sun protection products and the claims made relating to them. This recommendation establishes a series of guidelines regarding labeling and the information that must be provided to the consumer, in a clear and concise manner, for this type of product:
a. Sunscreen products should protect against both UVB and UVA radiation.
b. Sunscreen products must offer a minimum degree of protection against both UVB and UVA radiation.
c. The degree of protection must be measured by standardized and reproducible test methods, taking into account photodegradation. Preference should be given to *in vitro* test methods.
d. The efficacy of sunscreen products must be listed on the label by categories. The category of sun protection products must be indicated on the label at least as prominently as the sun protection factor.
e. Claims such as 100% protection against UV radiation (such as "sunscreen" or "total protection") should not be made; statements implying that it is not necessary to repeat the application of the product (such as "prevention throughout the day") should not be indicated in any case.
f. Sun protection products must carry warnings indicating that they do not constitute 100% protection, such as "Do not stay in the sun for a long time, even if you use a sun protection product. Keep babies and young children out of direct sunlight. Excessive sun exposure is a major health hazard".
g. Regarding the instructions for use, recommendations should be like "Apply before sun exposure. To maintain protection, repeat the application of the product frequently, especially after perspiring, bathing or drying". For consumers, the clearest and most effective way to apply this recommendation is to express the efficacy of a sunscreen product against sun exposure on the label using the acronym SPF, followed by the protection factor.

The acronym SPF stands for "Sun Protection Factor" and the number that accompanies this acronym indicates the ratio between the energy needed to produce a minimal erythematic response with application of the product to the skin and without it. Ultimately, the higher the SPF value, the more energy is required to produce the minimal erythematic response. Therefore, the higher the SPF value, the greater protection against UVB rays that product offers, since more energy is needed to produce that small burn or minimal erythematic response.

The cosmetic industry is making great efforts to provide consumers with products with an increasingly safe and effective sun protection factor, for which products must be designed that meet these requirements. It is known and recognized in the industry that for the design of formulations intended to protect humans from harmful effects, simulators are used to predict the degree of SPF protection, *in silico,* based on the percentage of UV filters used. Examples of this simulators include Digital Sun Protection Lab from BASF, Sunscreen Simulator from Abich or DSM Sunscreen Optimizer. These simulators are recognized within the industry and are used as reference values before testing the degree of sun protection using other techniques that will be explained below.

Normally, the SPF values obtained in an *in silico* method accurately predict the values obtained in an *in vitro* method.

The SPF factor is calculated by performing an *in vivo* test on volunteers conveniently standardized under the ISO 24444:2019 standard. This standard indicates all the parameters to be controlled to carry out the test: the selection of volunteers, and the way to carry out the photoprotection test, the interpretation of results and their statistical validity to give rigor to the results obtained.

Briefly, the test is carried out on a determined skin region of the volunteers. This specific and delimited region is exposed to ultraviolet radiation from a specific and known source without any protection and another region different from the previous one is exposed to ultraviolet radiation after the application of the product to be tested. The treated regions of each individual are visually evaluated for the presence of erythema in the area treated with the product to be tested and in the untreated area. From these exposures, MED (iu) values and MED (ip) values are derived, which are the minimum erythemal dose to a subject on untreated skin and the minimum erythema dose to a subject in the treated area, respectively. The SPF is calculated as the ratio between the MED (ip) and the MED (iu) of each individual. The average of all the SPF of the study volunteers gives the final SPF of the product.

Although work is under way to develop *in vitro* SPF test methods, ISO/CD 23698 and ISO/CD 23675, the only currently validated sun protection factor determination test is performed *in vivo* using ISO 24444: 2019.

According to the EU recommendation, another factor to consider is that the product must also offer protection against UVA rays. UVA protection can be determined by two methods: *in vivo* and *in vitro,* and as discussed for SPF, a prevalence of *in vitro* methods is preferred because they are not invasive with human volunteers.

ISO 24442:2011 *in vivo* method is analogous to the method indicated for SPF, but focusing only on the part of the UVA spectrum: with a UV lamp, an area of skin treated and another not treated with the product is irradiated in the UVA spectrum so as to obtain the minimum dose of pigmentation of the treated area (DMPPDp) and the minimum dose of persistent pigmentation (DMPPDnp) of the untreated area, respectively. Subsequently, the UVA protection factor of the evaluated product is obtained by calculating the quotient between the values of DMPPD and DMPPnp.

On the other hand, in the ISO 24443:2021 test we leave the volunteers aside and use a rough substrate where the product is applied and an absorbance curve of this product is made in the UV spectrum from which calculations can be made. for obtaining the in vitro protection factor in the UVA spectrum. Furthermore, these calculations can be correlated with the mentioned *In Vivo* Protection Factor.

Several reports are known in the art disclosing different effects of specific combinations of UV filters in cosmetic compositions.

WO2011146491 discloses a gel having a solar protection factor of at least 14 comprising at least about 50% by weight of a volatile hydrocarbon, a film former, a solvent comprising an ester group, a combination of ultraviolet A (UVA) and ultraviolet B (UVB) sunscreen agents, wherein the UVA sunscreen agent comprises 4-tert-butyl-4'-methoxydibenzoylmethane, wherein the PFA (protection factor of UVA) of the gel is at least about 5 or at least about 8, and wherein the gel is capable of drying within two minutes after topical application to skin.

WO2010129213 discloses compositions comprising UV blocking agents, a film forming polymer containing acid groups and a neutralizing agent; compositions and methods for increasing the SPF of a composition by neutralizing at least a portion of the acid groups of the film forming agent with the neutralizing agent.

EP3093005 discloses a cosmetic preparation containing a UV filter combination of a) 4-(tert-butyl)-4'-methoxydibenzoylmethane, b) 4,4'-[[6-[[4-[[(1,1-dimethylethyl)amino]carbonyl]-phenyl]amino]-1,3,5-triazine-2,4-diyl]di-imino]bis-, bis(2-ethylhexyl)benzoate (INCI: Diethylhexyl Butamido Triazone), c) 2,4-bis-{[4-(2-ethyl-hexyloxy)-2-hydroxy]phenyl}-6-(4-methoxyphenyl)-1,3,5-triazine (INCI: Bis-Ethylhexyloxyphenol Methoxyphenyl triazine) and d) one or more salicylates selected from the group of compounds 2-ethylhexyl 2-hydroxybenzoate (INCI: Ethylhexyl Salicylate) and 3,3,5-trimethylcyclohexyl 2-hydroxybenzoate (INCI: Homosalate).

EP3173129 discloses a cosmetic preparation comprising a) octyl salicylate (Ethylhexyl Salicylate), b) Titanium Dioxide in the crystal structure having a primary particle size of 2-100 nm of rutile.

WO2013113746 discloses a composition containing a photostabilized combination of Butyl Methoxydibenzoylmethane (BMDBM), Bis-Ethylhexyloxyphenol Methoxyphenyl Triazine (BEMT), and Methylene Bis- Benzotriazolyl Tetramethylbutylphenol (MBBT) wherein: ( i) - the BEMT/BMDBM mass ratio is greater than or equal to 1 and preferably greater than or equal to 1.5; (ii) - the content of BMDBM is comprised between 1% and 5% by weight with regard to the total weight of the composition; (iii) - the quantity of MBBT is comprised between 3% and 7% by weight with regard to the total weight of the composition, said combination containing no octocrylene, PABA or ethylhexyl methoxycinnamate, and a pharmaceutically or cosmetically acceptable excipient.

WO2007128840A2 discloses cosmetic preparations for the protection of the human skin and human hair against harmful effect of ultraviolet solar radiation containing the water soluble UVA absorbing substance Disodium Phenyl Dibenzimidazole Tetrasulfonate together with Troxerutin to quench the fluorescence of Disodium Phenyl Dibenzimidazole Tetrasulfonate.

US2016303020A1 discloses sunscreen compositions containing a primary UV-absorbing agent in an amount effective to absorb ultraviolet and about 5% or less of a visible light radiation agent that includes a continuous silica-containing coating.

EP2939710A1 discloses a cosmetic and/or pharmaceutical preparation comprising the soluble UVA absorbing substance Disodium Phenyl Dibenzimidazole Tetrasulfonate, wherein the preparation is free from the UVA filters selected from the group consisting of 2,4-bis[{(4-(2-ethylhexyloxy)-2-hydroxy}phenyl]--6-(4-methoxyphenyl)-1,3,5-triazine, (Tinosorb^{®}S), hexyl 2-(4-diethylamino-2-hydroxybenzoyl)benzoate (Uvinul^{®}A Plus), 2-hydroxy-4-methoxybenzophenone (Benzophenone-3, Oxybenzone, Neo Heliopan^{®}BB), Tris-Biphenyl Triazine (Tinosorb^{®}A2B) or mixtures thereof.The Applicant herein has surprisingly found a combination of UV filters yielding an unexpected technical effect, as will be described hereinbelow.

### SUMMARY OF THE INVENTION

One aspect of the present invention provides a use of a composition comprising a UV filter combination of Disodium 2,2'-(1,4-phenylene)bis(6-sulfo-1H-1,3-benzimidazole-4-sulfonate) (INCI: Disodium Phenyl Dibenzimidazole Tetrasulfonate), 2,2'-[6-(4-Methoxyphenyl)-1,3,5-triazine-2,4-diyl]bis{5-[(2-ethylhexyl)oxy]phenol} (INCI: Bis-Ethylhexyloxyphenol Methoxyphenyl Triazine), 4-[[4,6-bis[[4-(2-ethylhexoxy-oxomethyl)phenyl]amino]-1,3,5-triazin-2-yl]amino]benzoic acid 2-ethylhexyl ester (INCI: Ethylhexyl Triazone); and preferably and antioxidant, to increase the absorbance of a cosmetic preparation.

Another aspect of the present invention relates to a cosmetic preparation comprising a UV filter combination of Disodium 2,2'-(1,4-phenylene)bis(6-sulfo-1H-1,3-benzimidazole-4-sulfonate) (INCI: Disodium Phenyl Dibenzimidazole Tetrasulfonate), 2,2'-[6-(4-Methoxyphenyl)-1,3,5-triazine-2,4-diyl]bis{5-[(2-ethylhexyl)oxy]phenol} (INCI: Bis-Ethylhexyloxyphenol Methoxyphenyl Triazine), and 4-[[4,6-bis[[4-(2-ethylhexoxy-oxomethyl)phenyl]amino]-1,3,5-triazin-2-yl]amino]benzoic acid 2-ethylhexyl ester (INCI: Ethylhexyl Triazone), and at least three compounds selected from the group of the six compounds consisting of 2-ethylhexyl 2-hydroxybenzoate (INCI: Ethylhexyl Salicylate), 3-(4-tert-Butylphenyl)-1-(4-methoxyphenyl)propane-1,3-dione (INCI: Butyl Methoxydibenzoylmethane), Titanium Dioxide, 3,3,5-Trimethylcyclohexyl 2-hydroxybenzoate (INCI: Homosalate), 2-Ethylhexyl 2-cyano-3,3-diphenylprop-2-enoate (INCI: Octocrylene) and 3-Methylbutyl (2E)-3-(4-methoxyphenyl)prop-2-enoate (INCI: Isoamyl p-Methoxycinammate); preferably an antioxidant; and water in an amount of 30% to 40% with respect to the total amount of the preparation.

Another aspect of the present invention relates to a method for increasing the absorbance of a cosmetic preparation, the cosmetic preparation comprising a composition that comprises a UV filter combination of Disodium 2,2'-(1,4-phenylene)bis(6-sulfo-1H-1,3-benzimidazole-4-sulfonate) (INCI: Disodium Phenyl Dibenzimidazole Tetrasulfonate), 2,2'-[6-(4-Methoxyphenyl)-1,3,5-triazine-2,4-diyl]bis{5-[(2-ethylhexyl)oxy]phenol} (INCI: Bis-Ethylhexyloxyphenol Methoxyphenyl Triazine) and 4-[[4,6-bis[[4-(2-ethylhexoxy-oxomethyl)phenyl]amino]-1,3,5-triazin-2-yl]amino]benzoic acid 2-ethylhexyl ester (INCI: Ethylhexyl Triazone); and preferably an antioxidant; the method comprising the step of irradiating the cosmetic preparation with UV light.

Another aspect of the present invention relates to a dermatological preparation comprising the cosmetic preparation as described herein.

The present invention also relates to the use of the cosmetic photoactive composition described herein for the protection of the skin, preferably the human skin, against solar radiation.

Other features of the invention are disclosed in the detailed description of the invention.

### DETAILED DESCRIPTION OF THE INVENTION

The foregoing and other advantages and features will be more fully understood from the following detailed description of the invention. The examples disclosed herein are to be taken in an illustrative and not limitative way.

Unless otherwise indicated, all percentages relating to the content of a component or to a collection of components of the composition of the present invention refer to the weight percentage with respect to the total weight of the composition.

In the context of the present disclosure, the formulations "according to the invention", "preparation according to the invention" etc. always refer to the preparations, processes and uses according to the invention, i.e. also to preparations in which the uses according to the invention are realized as well as preparations with which the inventive method is realized.

Unless the context clearly requires otherwise, throughout the description and the claims, the words "comprise", "comprising", and the like are to be construed in an inclusive sense as opposed to an exclusive or exhaustive sense; that is to say, in the sense of "including, but not limited to".

In the context of the present disclosure, the terms "photoactive", "photoactivation" and the like are equivalent to the terms "fotoactive", "fotoactivation" and the like.

In the context of the present invention, solar radiation is to be understood as radiant energy from the sun. Solar radiation is largely optical radiation, being this optical radiation radiant energy within a broad region of the electromagnetic spectrum that includes ultraviolet (UV), visible (light) and infrared radiation. The wavelength of UV radiation (UVR) lies in the range of 100-400 nm, and is further subdivided into UVA (315-400 nm), UVB (280-315 nm) and UVC (100-280 nm). The UV component of terrestrial radiation from the midday sun comprises approximately 95% UVA and 5% UVB; UVC and most UVB are removed from extraterrestrial radiation by stratospheric ozone.

The cosmetic preparation is as defined in the present disclosure.

The cosmetic preparation according to the present invention is intended for protecting the skin and/or hair against solar radiation, in particular, ultraviolet radiation. To that end, the cosmetic preparation comprises a combination of UV filters in a cosmetically acceptable support.

The cosmetic preparation according to the present invention has the surprising effect of being photoactive, that is, the protection against solar radiation is increased after exposure of the cosmetic preparation according to the invention to solar radiation. Therefore, the term "photoactive" in the present application relates to the increase of the solar protection and/or UV protection of a preparation of the cosmetic preparation disclosed herein after solar and/or UV irradiation of the cosmetic preparation.

In other words, photoactivity may herein defined as the ability to absorb electromagnetic radiation, in particular UV radiation, by a substance or a composition that results in an increase of the absorbance after exposure to UV radiation as compared to the absorbance before exposure to UV radiation.

Therefore, a photoactive composition relates to a composition that yields values of absorbance (and, as a consequence, of solar protection) that are higher when exposed to UV radiation than before exposure.

The conditions by which this phenomenon takes place are defined in the *in vitro* method ISO 24443:2021 "Determination of sunscreen UVA photoprotection *in vitro".* Therefore, the absorbance in the context of the photoactivity shown by the composition according to the present invention can be determined by the mentioned ISO 24443:2021. Furthermore, there is a correlation of the absorbance with the Solar Protection Factor.

The conditions of the method disclosed in ISO 24443:2021 are briefly indicated as follows: the sample comprising the cosmetic preparation is applied to a rough substrate and an absorbance curve in the UV spectrum is performed on the sample (irradiation time: 20 minutes, energy 550 W/m2 and temperature of 30ºC). From the absorbance curve it is possible to obtain the *in vitro* protection factor in the UVA spectrum. Those calculations can be correlated to the SPF that is related to the protection factor in the UVB spectrum.

The afore-mentioned photoactivity is obtained by an specific combination of compounds in the cosmetic preparation according to the invention. In particular, this specific combination of compounds involves several UV filters and, preferably, at least one antioxidant.

A UV filter is understood in the art as ingredients or compounds that absorb or reflect the UV rays that are part of sun light. UV filters are used to absorb or reflect the UV rays that are contained in sun light or in artificial light. UV Filters can be used to protect the skin from the harmful effects of UV light (skin cancer and photo damage and wrinkling). UV Filters can also be used to protect products and their ingredients as well as packaging. UV Filters are sometimes used to protect hair color, especially for hair that has been dyed.

UV filters allowed in cosmetic products is commonly regulated/limited by national or supranational regulations. For instance, the Annex VI of the Regulation (EC) No 1223/2009 of the European Parliament and of the Council defines a list of UV filters allowed in cosmetic products in the European Union.

In the present application, the compounds are preferably named according to IUPAC rules. Compounds are also named according to the INCI (International Nomenclature of Cosmetic Ingredients) for better comprehension, when applicable.

Accordingly, the cosmetic preparation according to the present invention comprises a UV filter combination of Disodium 2,2'-(1,4-phenylene)bis(6-sulfo-1H-1,3-benzimidazole-4-sulfonate) (INCI: Disodium Phenyl Dibenzimidazole Tetrasulfonate), and at least five compounds selected from the group of the eight compounds consisting of 2-ethylhexyl 2-hydroxybenzoate (INCI: Ethylhexyl Salicylate), 2,2'-[6-(4-Methoxyphenyl)-1,3,5-triazine-2,4-diyl]bis{5-[(2-ethylhexyl)oxy]phenol} (INCI: Bis-Ethylhexyloxyphenol Methoxyphenyl Triazine), 4-[[4,6-bis[[4-(2-ethylhexoxy-oxomethyl)phenyl]amino]-1,3,5-triazin-2-yl]amino]benzoic acid 2-ethylhexyl ester (INCI: Ethylhexyl Triazone), 3-(4-tert-Butylphenyl)-1-(4-methoxyphenyl)propane-1,3-dione (INCI: Butyl Methoxydibenzoylmethane), Titanium Dioxide, 3,3,5-Trimethylcyclohexyl 2-hydroxybenzoate (INCI: Homosalate), 2-Ethylhexyl 2-cyano-3,3-diphenylprop-2-enoate (INCI: Octocrylene) and 3-Methylbutyl (2E)-3-(4-methoxyphenyl)prop-2-enoate (INCI: Isoamyl p-Methoxycinammate); and preferably an antioxidant.

The cosmetic preparation according to the present invention also discloses a UV filter combination of Disodium Phenyl Dibenzimidazole Tetrasulfonate, and seven compounds selected from the group of the eight compounds consisting of Ethylhexyl Salicylate, Bis-Ethylhexyloxyphenol Methoxyphenyl Triazine, Ethylhexyl Triazone, Butyl Methoxydibenzoylmethane, Titanium Dioxide, Homosalate, Octocrylene and Isoamyl p-Methoxycinammate.

Another cosmetic preparation according to the present invention comprises a UV filter combination of Disodium Phenyl Dibenzimidazole Tetrasulfonate, Ethylhexyl Salicylate, Bis-Ethylhexyloxyphenol Methoxyphenyl Triazine, Ethylhexyl Triazone, Butyl Methoxydibenzoylmethane, Titanium Dioxide, Homosalate, Octocrylene and Isoamyl p-Methoxycinammate).

The cosmetic preparation of the present invention may also comprise a UV filter combination of Disodium Phenyl Dibenzimidazole Tetrasulfonate, Ethylhexyl Salicylate, Bis-Ethylhexyloxyphenol Methoxyphenyl Triazine, Ethylhexyl Triazone, Butyl Methoxydibenzoylmethane and Titanium Dioxide.

The cosmetic preparation of the present invention may also comprise a UV filter combination consisting of Disodium Phenyl Dibenzimidazole Tetrasulfonate, Ethylhexyl Salicylate, Bis-Ethylhexyloxyphenol Methoxyphenyl Triazine, Ethylhexyl Triazone, Butyl Methoxydibenzoylmethane and Titanium Dioxide.

The cosmetic preparation may further comprise Homosalate. Preferably, the cosmetic preparation may further comprise Octocrylene. More preferably, the cosmetic preparation may further comprise Isoamyl p-Methoxycinammate.

The cosmetic preparation may be further characterized in that the UV filters combination is in an amount in the range from 2% to 40% by weight with respect to the total preparation, preferably, from 5% to 30%, preferably from 5% to 25%, more preferably, from 10% to 25%.

The antioxidant in the cosmetic preparation according to the present invention is preferably selected from the group comprising Theobroma cacao seed extract, Tocopherol, Tocopheryl acetate, Ascorbyl palmitate and combinations thereof. The antioxidant is preferably a combination of Tocopheryl acetate and Theobroma cacao seed extract. More preferably, the antioxidant is Theobroma cacao seed extract. The antioxidants according to the present invention may have a physiological action once applied e.g. in the skin and/or the function of preserving the cosmetic preparation from oxidation.

The antioxidant or combination of antioxidants in the cosmetic preparation according to the present invention is preferably in an amount in the range from 0,001% to 5% by weight with respect to the total preparation, preferably, from 0,001% to 3%, preferably from 0,01% to 3%, preferably from 0,01% to 2%, more preferably, from 0,01% to 1,5%, even more preferably from 0,05 to 1,5%. Moreover, Theobroma cacao seed extract is preferably in an amount in the range from 0,001% to 0,1%, more preferably from 0,01% to 0,02%.

The cosmetic preparation according to the present invention may further comprise an oil selected from the group of the esters C12-13 alkyl esters of 2-hydroxypropanoic acid (INCI: C12-13 Alkyl Lactate), C12-15 alkyl esters of benzoic acid (INCI: C12-15 Alkyl Benzoate), 2-phenylethyl benzoate (INCI: Phenethyl Benzoate), Dodecyl/Tetradecyl Benzoate (INCI: Lauryl/Myristyl Benzoate), Dipropan-2-yl-hexanediaote (INCI: Diisopropyl Adipate), 2-butyloctyl 2-hydroxybenzoate (INCI: Butyloctyl Salicylate), Dioctyl carbonate (INCI: Dicaprylyl Carbonate), 2-[1-(2-hexyldodecoxy)propan-2-yloxy]ethyl acetate (INCI: Propylene Glycol Isoceteth-3 Acetate), 3-octanoyloxybutyl/3-decanoyloxybutyl octanoate (INCI: Butylene Glycol Dicaprylate/Dicaprate), Dibutyl hexanedioate (INCI: Dibutyl Adipate), Propan2-yl tetradeconate (INCI: Isopropyl Myristate), Dodecyl decanoate (INCI: Coco Caprylate), Dodecyl decanoate/octanoate (INCI: Coco-Caprylate/Caprate), 2-propylheptyl octanoate (INCI: Propylheptyl Caprylate), (3-heptanoyloxy-2,2-dimethylpropyl) heptanoate (INCI: Neopentyl Glycol Diheptanoate), Diisopropyl Sebacate and combinations thereof. Preferably, the oil is selected from Butylene Glycol Dicaprylate/Dicaprate, Dibutyl Adipate, Coco-Caprylate/Caprate and combinations thereof. More preferably, the oil is selected from Butylene Glycol Dicaprylate/Dicaprate, Dibutyladipate and combinations thereof.

The oil or combination of oils in the cosmetic preparation according to the present invention are preferably in an amount in the range from 5% to 45% by weight with respect to the total preparation, preferably, from 10% to 40%, preferably from 15% to 35%, preferably from 20% to 30%, more preferably, from 21% to 27%.

The cosmetic preparation according to the present invention is preferably characterized in that the preparation is in the form of an oil-in-water (O/W) emulsion, a water-in-oil (W/O) emulsion, silicone-in-water (Si/W) emulsion, a water-in-silicone (W/Si) emulsion, a stick or a polymeric emulsion. It is advantageous according to the invention if the preparation according to the invention is in the form of an emulsion. In this case, it is preferred, according to the invention, that the preparation is in the form of an oil-in-water emulsion (O/W emulsion). According to the invention, the O/W emulsion is more preferably in the form of a lotion.

The cosmetic preparation according to the present invention may comprise water, preferably in an amount in the range from 20% to 60% by weight with respect to the total amount of the preparation, preferably from 30% to 45%, more preferably from 35% to 40%.

Therefore, the present disclosure also relates to a cosmetic preparation comprising a UV filter combination of Disodium 2,2'-(1,4-phenylene)bis(6-sulfo-1H-1,3-benzimidazole-4-sulfonate) (INCI: Disodium Phenyl Dibenzimidazole Tetrasulfonate), 2,2'-[6-(4-Methoxyphenyl)-1,3,5-triazine-2,4-diyl]bis{5-[(2-ethylhexyl)oxy]phenol} (INCI: Bis-Ethylhexyloxyphenol Methoxyphenyl Triazine), and 4-[[4,6-bis[[4-(2-ethylhexoxy-oxomethyl)phenyl]amino]-1,3,5-triazin-2-yl]amino]benzoic acid 2-ethylhexyl ester (INCI: Ethylhexyl Triazone), and at least three compounds selected from the group of the eight compounds consisting 2-ethylhexyl 2-hydroxybenzoate (INCI: Ethylhexyl Salicylate), 3-(4-tert-Butylphenyl)-1-(4-methoxyphenyl)propane-1,3-dione (INCI: Butyl Methoxydibenzoylmethane), Titanium Dioxide, 3,3,5-Trimethylcyclohexyl 2-hydroxybenzoate (INCI: Homosalate), 2-Ethylhexyl 2-cyano-3,3-diphenylprop-2-enoate (INCI: Octocrylene) and 3-Methylbutyl (2E)-3-(4-methoxyphenyl)prop-2-enoate (INCI: Isoamyl p-Methoxycinammate); preferably an antioxidant; and water in an amount of 30% to 40% with respect to the total amount of the preparation.

The present disclosure relates to a cosmetic preparation comprising a UV filter combination comprising Disodium 2,2'-(1,4-phenylene)bis(6-sulfo-1H-1,3-benzimidazole-4-sulfonate) (INCI: Disodium Phenyl Dibenzimidazole Tetrasulfonate), 2,2'-[6-(4-Methoxyphenyl)-1,3,5-triazine-2,4-diyl]bis{5-[(2-ethylhexyl)oxy]phenol} (INCI: Bis-Ethylhexyloxyphenol Methoxyphenyl Triazine), and 4-[[4,6-bis[[4-(2-ethylhexoxy-oxomethyl)phenyl]amino]-1,3,5-triazin-2-yl]amino]benzoic acid 2-ethylhexyl ester (INCI: Ethylhexyl Triazone), 2-ethylhexyl 2-hydroxybenzoate (INCI: Ethylhexyl Salicylate), 3-(4-tert-Butylphenyl)-1-(4-methoxyphenyl)propane-1,3-dione (INCI: Butyl Methoxydibenzoylmethane) and Titanium Dioxide; preferably an antioxidant; and water in an amount of 30% to 40% with respect to the total amount of the preparation.

The inventors of the present application found that the UV filter Disodium Phenyl Dibenzimidazole Tetrasulfonate was essential for the fotoactivation to take place. Moreover, the combination of Bis-Ethylhexyloxyphenol Methoxyphenyl Triazine together with Ethylhexyl Triazone is also essential for the fotoactivation.

In particular, a combination of 8 filters not comprising Disodium Phenyl Dibenzimidazole Tetrasulfonate yielded no fotoactivation at all.

In particular, when the UV filters Bis-Ethylhexyloxyphenol Methoxyphenyl Triazine and Ethylhexyl Triazone were eliminated from a cosmetic preparation comprising Disodium Phenyl Dibenzimidazole Tetrasulfonate, no fotoactivation was obtained, and the values of SPF were dramatically reduced.

Therefore, the combination of the following 6 filters were found to be especially convenient in terms of fotoactivation and SPF protection: Disodium Phenyl Dibenzimidazole Tetrasulfonate, Bis-Ethylhexyloxyphenol Methoxyphenyl Triazine, Ethylhexyl Triazone, Butyl Methoxydibenzoylmethane, Titanium Dioxide and Ethylhexyl Salicylate.

The cosmetic preparation according to the present invention may further comprise a polyglyceryl-based emulsifier. Such polyglyceryl-based emulsifier may preferably be selected from the group comprising [(2R)-2-[(2R,3R,4S)-3,4-dihydroxyoxolan-2-yl]-2-hydroxyethyl] (Z)-octadec-9-enoate (INCI: Sorbitan Oleate), 2,3-dihydroxypropyl octadecanoate (INCI: Glyceryl Stearate), 2,3-dihydroxypropyl (Z)-octadec-9-enoate (INCI: Glyceryl Oleate), 1,2,3-propanetriol dodecanoate/1,4-butanedioate esters (INCI: Polyglyceryl-4-laurate/succinate) and combinations thereof.

The polyglyceryl-based emulsifier or combination of the polyglyceryl-based emulsifiers in the cosmetic preparation according to the present invention is preferably in an amount in the range from 0,001% to 10% by weight with respect to the total amount of the preparation, preferably from 0,005% to 5%, more preferably from 0,01% to 2,5%.

The cosmetic preparation according to the present invention may further comprise a surfactant. Such surfactant may preferably be selected from the group comprising (3R,4S,5S,6R)-2-dodecoxy-6-(hydroxymethyl)oxane-3,4,5-triol (INCI: Coco-Glucoside), disodium 4-dodecoxy-4-oxo-2-sulfonatobutanoate / disodium 4-dodecoxy-4-oxo-2-sulfonatobutanoate (INCI: Disodium Lauryl Sulfosuccinate) and combinations thereof.

The surfactant or combination of surfactants in the cosmetic preparation according to the present invention is preferably in the range from 0,01% to 1% by weight with respect to the total amount of the preparation, preferably from 0,05% to 0,5%, preferably from 0,05% to 0,25% more preferably from 0,05% to 0,2%.

The cosmetic preparation according to the present invention may further comprise a preservative selected from the group of 3-phenylpropan-1-ol (INCI: Phenlypropanol), Propane-1,3-diol (INCI: Propanediol), Octane-1,2-diol (INCI: Caprylyl Glycol), hexane-1,2-diol (INCI: 1,2-Hexanediol), Pentane-1,2-diol (INCI: Pentylene Glycol), 1,3-butanediol (INCI: Butylene Glycol), 3-(2-ethylhexoxy)propane-1,2-diol (INCI: Ethylhexylglycerin), 2-phenoxyethanol (INCI: Phenoxyethanol) and combinations thereof. Preferably, the preservatives are selected from the group of Phenoxyethanol, Ethylhexylglycerin, Phenylpropanol, 1,2-Hexanediol and combinations thereof.

The preservative or combination of preservatives in the cosmetic preparation according to the present invention is preferably in an amount in the range from 0,05% to 5% by weight with respect to the total preparation, preferably, from 0,05% to 2,5%, preferably from 0,05% to 1,5%, preferably from 0,1% to 1,5%, more preferably, from 0,01% to 1.

The cosmetic preparation of the present disclosure preferably does not comprise Troxerutin or a fluorescence quencher of Disodium Phenyl Benzimidazole Tetrasulfonic acid or Disodium Phenyl Benzimidazole Tetrasulfonate. The Applicant has found that Troxerutin, a substance with the ability to prevent fluorescence of Disodium Phenyl Benzimidazole Tetrasulfonate and disclosed in prior art document WO207128840A2, inhibits the photoactive properties of the cosmetic preparation disclosed herein.

The inventors of the present application surprisingly found that the combination of UV filters herein disclosed provided an increase of the protection ability of the composition against UV radiation (UVA and UVB) after exposure.

Moreover, the values of UV absorbance and SPF obtained *in vitro* and *in vivo* were significantly higher than the values predicted by the *in silico* models.

The present disclosure also relates to a use of the composition as described herein to increase the absorbance of a cosmetic preparation. The absorbance of the cosmetic preparation might be measured according to ISO 24443:2021.

Accordingly, the the present disclosure relates to the use of a composition comprising a UV filter combination of Disodium 2,2'-(1,4-phenylene)bis(6-sulfo-1H-1,3-benzimidazole-4-sulfonate) (INCI: Disodium Phenyl Dibenzimidazole Tetrasulfonate), 2,2'-[6-(4-Methoxyphenyl)-1,3,5-triazine-2,4-diyl]bis{5-[(2-ethylhexyl)oxy]phenol} (INCI: Bis-Ethylhexyloxyphenol Methoxyphenyl Triazine), 4-[[4,6-bis[[4-(2-ethylhexoxy-oxomethyl)phenyl]amino]-1,3,5-triazin-2-yl]amino]benzoic acid 2-ethylhexyl ester (INCI: Ethylhexyl Triazone); and preferably an antioxidant, to increase the absorbance of a cosmetic preparation.

It has been found, as disclosed herein, that the combination of the UV filters Disodium Phenyl Dibenzimidazole Tetrasulfonate, Bis-Ethylhexyloxyphenol Methoxyphenyl Triazine and Ethylhexyl Triazone is essential for the photoactivation to take place, i.e., for the cosmetic preparation comprising such a combination to increase the absorbance thereof.

In particular, the present disclosure relates to the use of a composition comprising a UV filter combination of Disodium 2,2'-(1,4-phenylene)bis(6-sulfo-1H-1,3-benzimidazole-4-sulfonate) (INCI: Disodium Phenyl Dibenzimidazole Tetrasulfonate), 2,2'-[6-(4-Methoxyphenyl)-1,3,5-triazine-2,4-diyl]bis{5-[(2-ethylhexyl)oxy]phenol} (INCI: Bis-Ethylhexyloxyphenol Methoxyphenyl Triazine), 4-[[4,6-bis[[4-(2-ethylhexoxy-oxomethyl)phenyl]amino]-1,3,5-triazin-2-yl]amino]benzoic acid 2-ethylhexyl ester (INCI: Ethylhexyl Triazone), and at least three compounds selected from the group of the six compounds consisting of 2-ethylhexyl 2-hydroxybenzoate (INCI: Ethylhexyl Salicylate), 3-(4-tert-Butylphenyl)-1-(4-methoxyphenyl)propane-1,3-dione (INCI: Butyl Methoxydibenzoylmethane), Titanium Dioxide, 3,3,5-Trimethylcyclohexyl 2-hydroxybenzoate (INCI: Homosalate), 2-Ethylhexyl 2-cyano-3,3-diphenylprop-2-enoate (INCI: Octocrylene) and 3-Methylbutyl (2E)-3-(4-methoxyphenyl)prop-2-enoate (INCI: Isoamyl p-Methoxycinammate); and preferably an antioxidant, to increase the absorbance of a cosmetic preparation.

In particular, the present disclosure relates to the use of a composition comprising a UV filter combination comprising Disodium 2,2'-(1,4-phenylene)bis(6-sulfo-1H-1,3-benzimidazole-4-sulfonate) (INCI: Disodium Phenyl Dibenzimidazole Tetrasulfonate), 2,2'-[6-(4-Methoxyphenyl)-1,3,5-triazine-2,4-diyl]bis{5-[(2-ethylhexyl)oxy]phenol} (INCI: Bis-Ethylhexyloxyphenol Methoxyphenyl Triazine), and 4-[[4,6-bis[[4-(2-ethylhexoxy-oxomethyl)phenyl]amino]-1,3,5-triazin-2-yl]amino]benzoic acid 2-ethylhexyl ester (INCI: Ethylhexyl Triazone), 2-ethylhexyl 2-hydroxybenzoate (INCI: Ethylhexyl Salicylate), 3-(4-tert-Butylphenyl)-1-(4-methoxyphenyl)propane-1,3-dione (INCI: Butyl Methoxydibenzoylmethane) and Titanium Dioxide; and preferably an antioxidant, to increase the absorbance of a cosmetic preparation.

Preferably, the use of the composition to increase the absorbance of a cosmetic preparation of the present disclosure may comprise a composition that comprises water, in an amount in the range from 30% to 45%, more preferably from 30% to 40%, even more preferably from 35% to 40% by weight with respect to the total amount of the preparation.

The amount of water in the composition as defined herein is relevant as to the photoactivity and the SPF of the cosmetic preparation. The inventors have surprisingly found that the range above described advantageously provide higher values of photoactivation and solar photoprotection. When the amount of water is higher, the photoactivation and the SPF decrease.

Preferably, the use of the composition to increase the absorbance of a cosmetic preparation of the present disclosure may comprise a composition that does not comprise Troxerutin or a fluorescence quencher of Disodium Phenyl Dibenzimidazole Tetrasulfonic Acid or Disodium Phenyl Dibenzimidazole Tetrasulfonate.

The present disclosure also relates to a method for increasing the absorbance of the cosmetic preparation herein disclosed, the method comprising the step of irradiating the cosmetic preparation with UV light. Irradiation of the cosmetic preparation might be carried out as described in ISO 24442:2011 and/or ISO 24443:2021.

Accordingly, the present disclosure relates to a method for increasing the absorbance of a cosmetic preparation, the cosmetic preparation comprising a composition that comprises a UV filter combination of Disodium 2,2'-(1,4-phenylene)bis(6-sulfo-1H-1,3-benzimidazole-4-sulfonate) (INCI: Disodium Phenyl Dibenzimidazole Tetrasulfonate), 2,2'-[6-(4-Methoxyphenyl)-1,3,5-triazine-2,4-diyl]bis{5-[(2-ethylhexyl)oxy]phenol} (INCI: Bis-Ethylhexyloxyphenol Methoxyphenyl Triazine), and 4-[[4,6-bis[[4-(2-ethylhexoxy-oxomethyl)phenyl]amino]-1,3,5-triazin-2-yl]amino]benzoic acid 2-ethylhexyl ester (INCI: Ethylhexyl Triazone), and preferably an antioxidant; the method comprising the step of irradiating the cosmetic preparation with UV light.

In particular, the present disclosure relates to a method for increasing the absorbance of a cosmetic preparation, the cosmetic preparation comprising a composition, the composition comprising a UV filter combination of Disodium 2,2'-(1,4-phenylene)bis(6-sulfo-1H-1,3-benzimidazole-4-sulfonate) (INCI: Disodium Phenyl Dibenzimidazole Tetrasulfonate), 2,2'-[6-(4-Methoxyphenyl)-1,3,5-triazine-2,4-diyl]bis{5-[(2-ethylhexyl)oxy]phenol} (INCI: Bis-Ethylhexyloxyphenol Methoxyphenyl Triazine), and 4-[[4,6-bis[[4-(2-ethylhexoxy-oxomethyl)phenyl]amino]-1,3,5-triazin-2-yl]amino]benzoic acid 2-ethylhexyl ester (INCI: Ethylhexyl Triazone), and at least three compounds selected from the group of the eight compounds consisting 2-ethylhexyl 2-hydroxybenzoate (INCI: Ethylhexyl Salicylate), 3-(4-tert-Butylphenyl)-1-(4-methoxyphenyl)propane-1,3-dione (INCI: Butyl Methoxydibenzoylmethane), Titanium Dioxide, 3,3,5-Trimethylcyclohexyl 2-hydroxybenzoate (INCI: Homosalate), 2-Ethylhexyl 2-cyano-3,3-diphenylprop-2-enoate (INCI: Octocrylene) and 3-Methylbutyl (2E)-3-(4-methoxyphenyl)prop-2-enoate (INCI: Isoamyl p-Methoxycinammate); and preferably an antioxidant; the method comprising the step of irradiating the cosmetic preparation with UV light.

Preferably, the method for increasing the absorbance of a cosmetic preparation comprises a UV filter combination that comprises Disodium 2,2'-(1,4-phenylene)bis(6-sulfo-1H-1,3-benzimidazole-4-sulfonate) (INCI: Disodium Phenyl Dibenzimidazole Tetrasulfonate), 2,2'-[6-(4-Methoxyphenyl)-1,3,5-triazine-2,4-diyl]bis{5-[(2-ethylhexyl)oxy]phenol} (INCI: Bis-Ethylhexyloxyphenol Methoxyphenyl Triazine), and 4-[[4,6-bis[[4-(2-ethylhexoxy-oxomethyl)phenyl]amino]-1,3,5-triazin-2-yl]amino]benzoic acid 2-ethylhexyl ester (INCI: Ethylhexyl Triazone), 2-ethylhexyl 2-hydroxybenzoate (INCI: Ethylhexyl Salicylate), 3-(4-tert-Butylphenyl)-1-(4-methoxyphenyl)propane-1,3-dione (INCI: Butyl Methoxydibenzoylmethane) and Titanium Dioxide.

In the present disclosure, the UV light irradiating the cosmetic preparation is UVA and/or UVB light.

Preferably, the method for increasing the absorbance of a cosmetic preparation as in the present disclosure does not comprise Troxerutin or a fluorescence quencher of Disodium Phenyl Benzimidazole Tetrasulfonic Acid or Disodium Phenyl Benzimidazole Tetrasulfonate.

The preparation according to the present invention may also comprise other readily available ingredients for the skilled in the art in the field of cosmetics, such as stabilizers, parfum, chelating agents, pH regulators, texturizing, humectants, excipients, surfactants, polymers, among others.

Stabilizers may preferably be selected from Xanthan Gum, Magnesium Aluminum Silicate and combinations thereof; chelants may preferably be selected from Disodium EDTA, Sodium Phytate and combinations thereof; pH regulators may be selected from Citric Acid and Arginine; texturizing is preferably Silica; humectants are preferably selected from Butylene Glycol, Propanediol and combinations thereof; excipients are preferably selected from Lecithin, Alcohol and combinations thereof; surfactants are preferably selected from the group of Disodium Lauryl Sulfosuccinate, Coco-Glucoside and combinations thereof; polymer is preferably Acrylate/C12-22 Alkyl Methacrylate Copolymer.

The present invention also discloses a dermatological preparation comprising the preparation disclosed herein.

The present invention also relates to a method to obtain the preparation disclosed therein by mixing the several components comprising the preparation according to the present invention.

### EXAMPLES

### Example 1: Preparation compositions

Several preparations according to the invention were prepared. The following table accounts for the typical ingredients present in the preparation and their corresponding function.

### Preparation 1 (A-J)

**Table 1. Base composition for several compositions (A-J) according with the present invention.**

| **Function** | **Component (INCl name)** |
|---|---|
| Solvent | Aqua |
| Gelling agent / Emulsion stabilizer | Disteardimonium Hectorite, Sodium Chloride, Magnesium Sulfate |
| Humectant | Butylene glycol |
| Chelant | Disodium EDTA |
| Preservative | Ethylhexylglycerin, Phenoxyethanol, Sodium Benzoate, Potassium sorbate |
| pH regulator | Arginine |
| Antioxidant | Tocopheryl acetate, Theobroma cacao seed extract |
| Parfum | Parfum |
| Skin conditioner | Cichorium Intybus Root Extract |
| Texturizer | Silica |
| Emulsifier | PEG-30 Dipolyhydroxystearate, PEG-10 Dimethicone |
| UV filters | A: Disodium Phenyl Dibenzimidazole Tetrasulfonate, Bis-Ethylhexyloxyphenol Methoxyphenyl Triazine, Ethylhexyl Salicylate, Ethylhexyl triazone, Titanium Dioxide, Butyl-methoxydibenzoylmethane, Octocrylene, |
| | Homosalate and Isoamyl p-Methoxycinammate. |
| | B: Disodium Phenyl Dibenzimidazole Tetrasulfonate, Bis-Ethylhexyloxyphenol Methoxyphenyl Triazine, Ethylhexyl Salicylate, Ethylhexyl triazone, Titanium Dioxide, Butyl-methoxydibenzoylmethane, Octocrylene and Homosalate. |
| | C: Disodium Phenyl Dibenzimidazole Tetrasulfonate, Bis-Ethylhexyloxyphenol Methoxyphenyl Triazine, Ethylhexyl Salicylate, Ethylhexyl triazone, Butyl-methoxydibenzoylmethane, Octocrylene, Homosalate and Isoamyl p-Methoxycinammate. |
| | D: Disodium Phenyl Dibenzimidazole Tetrasulfonate, Bis-Ethylhexyloxyphenol Methoxyphenyl Triazine, Ethylhexyl Salicylate, Titanium Dioxide, Butyl-methoxydibenzoylmethane, Octocrylene, Homosalate and Isoamyl p-Methoxycinammate. |
| | E: Disodium Phenyl Dibenzimidazole Tetrasulfonate, Bis-Ethylhexyloxyphenol Methoxyphenyl Triazine, Ethylhexyl Salicylate, Ethylhexyl triazone, Titanium Dioxide, Octocrylene, Homosalate and Isoamyl p-Methoxycinammate. |
| | F: Disodium Phenyl Dibenzimidazole Tetrasulfonate, Ethylhexyl Salicylate, Ethylhexyl triazone, Titanium Dioxide, Butyl-methoxydibenzoylmethane, Octocrylene, Homosalate and Isoamyl p-Methoxycinammate. |
| | G: Disodium Phenyl Dibenzimidazole Tetrasulfonate, Bis-Ethylhexyloxyphenol Methoxyphenyl Triazine, Ethylhexyl triazone, Titanium Dioxide, Butyl-methoxydibenzoylmethane, |
| | Octocrylene, Homosalate and Isoamyl p-Methoxycinammate. |
| | H: Disodium Phenyl Dibenzimidazole Tetrasulfonate, Bis-Ethylhexyloxyphenol Methoxyphenyl Triazine, Ethylhexyl Salicylate, Ethylhexyl triazone, Titanium Dioxide, Butyl-methoxydibenzoylmethane, Octocrylene and Isoamyl p-Methoxycinammate. |
| | I: Disodium Phenyl Dibenzimidazole Tetrasulfonate, Bis-Ethylhexyloxyphenol Methoxyphenyl Triazine, Ethylhexyl Salicylate, Ethylhexyl triazone, Titanium Dioxide, Butyl-methoxydibenzoylmethane, Homosalate and Isoamyl p-Methoxycinammate. |
| | J: Bis-Ethylhexyloxyphenol Methoxyphenyl Triazine, Ethylhexyl Salicylate, Ethylhexyl triazone, Titanium Dioxide, Butyl-methoxydibenzoylmethane, Octocrylene, Homosalate and Isoamyl p-Methoxycinammate. |
| | |

The following two tables 2 and 3 disclose the specific amounts for two different preparations according to the present invention having protection factors of 50₊ and 30. These two preparations comprise the following 6 UV filters: Disodium Phenyl Dibenzimidazole Tetrasulfonate, Bis-Ethylhexyloxyphenol Methoxyphenyl Triazine, Ethylhexyl Salicylate, Ethylhexyl Triazone, Titanium Dioxide and Butyl-Methoxydibenzoylmethane. Preparation K has a UV filter amount of 20% by weight with respect to the total preparation, showing a solar protection factor above 50 (50₊), whereas Preparation L has a UV filter amount of 12%, showing a solar protection factor of above 30.

### Preparation K

**Table 2. Components of the specific preparation K according to the present invention including their relative proportions.**

| **Component (INCl name or function)** | **% w/w** |
|---|---|
| Aqua (Solvent) | 38,17 |
| Stabilizer / Viscosity regulator | 0,935 |
| Parfum | 0,1 |
| Chelant | 0,2 |
| pH regulator | 0,9 |
| Texturizer | 4,72 |
| Preservative | 1,5 |
| Humectant | 3,55 |
| Emollients | 21,8 |
| Bis-Ethylhexyloxyphenol Methoxyphenyl Triazine (UV Filter) | 6,2 |
| Ethylhexyl Salicylate (UV Filter) | 5,0 |
| Ethylhexyl triazone (UV Filter) | 4,0 |
| Titanium Dioxide (UV Filter) | 1,3 |
| Disodium Phenyl Dibenzimidazole Tetrasulfonate (UV Filter) | 1,0 |
| Butyl methoxydibenzoylmethane (UV Filter) | 3,0 |
| Emulsifiers | 4,5375 |
| Polymer | 1,68 |
| Antioxidant | 1,0585 |
| Surfactant | 0,254 |
| Active | 0,055 |
| Excipient | 0,04 |

### Preparation L

**Table 3. Components of the specific preparation L according to the present invention including their relative proportions.**

| **Component (INCl name or function)** | **% w/w** |
|---|---|
| Aqua (Solvent) | 40,677 |
| Stabilizer / Viscosity regulator | 1,1 |
| Parfum | 0,1 |
| Chelant | 0,2 |
| pH regulator | 0,9 |
| Texturizer | 4,035 |
| Preservative | 1,5 |
| Humectant | 3,55 |
| Emollients | 27,9 |
| Bis-Ethylhexyloxyphenol Methoxyphenyl Triazine (UV Filter) | 3,798 |
| Ethylhexyl Salicylate (UV Filter) | 3,0 |
| Ethylhexyl triazone (UV Filter) | 2,4 |
| Titanium Dioxide (UV Filter) | 0,765 |
| Disodium Phenyl Dibenzimidazole Tetrasulfonate (UV Filter) | 0,65 |
| Butyl methoxydibenzoylmethane (UV Filter) | 1,8 |
| Emulsifiers | 4,5375 |
| Polymer | 1,68 |
| Antioxidant | 1,0925 |
| Surfactant | 0,254 |
| Active | 0,055 |
| Excipient | 0,04 |

### Preparations M, N, O and P

**Table 4. Components of cosmetic preparations M, N, O and P. M is a preparation according to the invention, while preparations N, O and P are comparative compositions.**

| | **M** | **N** | **O** | **P** |
|---|---|---|---|---|
| **Component (INCl name or function)** | **%w/w** | **%w/w** | **%w/w** | **%w/w** |
| Aqua (Solvent) | 39,84265 | 49,97955 | 52,26955 | 38,30015 |
| Emollients | 21,8 | 21,8 | 21,8 | 29,8 |
| Bis-Ethylhexyloxyphenol Methoxyphenyl Triazine (UV Filter) | 6,198 | 6,198 | 6,198 | 2,178 |
| Ethylhexyl Salicylate (UV Filter) | 5 | 5 | 5 | 5 |
| Texturizer | 4,725 | 0 | 0 | 4,725 |
| Ethylhexyl triazone (UV Filter) | 4 | 4 | 4 | 0 |
| Butyl methoxydibenzoylmethane (UV Filter) | 3 | 3 | 3 | 3 |
| Humectant | 3 | 0 | 0 | 3,55 |
| Emulsifiers | 4,515 | 5,015 | 4,225 | 4,515 |
| Polymer | 1,683 | 1,683 | 1,683 | 1,683 |
| Titanium Dioxide (UV Filter) | 1,275 | 1,275 | 1,275 | 1,275 |
| Disodium Phenyl Dibenzimidazole Tetrasulfonate (UV Filter) | 1 | 1 | 0 | 1 |
| Antioxidant | 0 | 0 | 0 | 1,0125 |
| Preservatives | 1,5069 | 0 | 0 | 1,5069 |
| Stabilizer / Viscosity regulator | 0,95 | 0 | 0 | 0,95 |
| pH regulator | 0,897 | 0,797 | 0,297 | 0,897 |
| Chelant | 0,20 | 0 | 0 | 0,20 |
| Surfactant | 0,25245 | 0,25245 | 0,25245 | 0,25245 |
| Parfum | 0,1 | 0 | 0 | 0,1 |
| Active | 0,055 | 0 | 0 | 0,055 |
| TOTAL | 100 | 100 | 100 | 100 |

### Example 2: Activity of the compositions

The preparations according to the invention in the form of an O/W emulsion were deposited onto Poly(methyl methacrylate) (PMMA) plates and submitted to solar light and UV-A light for analysis of their protection parameters. Four measures were performed with a Kontron 933 spectrophotometer equipped with a sphere of integration to determine the sun protection factors

Two parameters, Solar protection factor and UV-A protection were measured before and after irradiation as disclosed hereinabove. The values of SPF and UV-A before and after UV irradiation allowed the calculation of the percentage of photoactivation of the preparations. The results are summarized in the following tables 5, 6 and 7.

Several preparations according to Table 1 were envisaged with different UV filters as follows (and included in Table 1):
Preparation A: 9 UV filters: Disodium Phenyl Dibenzimidazole Tetrasulfonate, Ethylhexyl Salicylate, Bis-Ethylhexyloxyphenol Methoxyphenyl Triazine, Ethylhexyl Triazone, Butyl Methoxydibenzoylmethane, Titanium Dioxide, Homosalate, Octocrylene and Isoamyl p-Methoxycinammate.
Preparation B: 8 UV filters: Disodium Phenyl Dibenzimidazole Tetrasulfonate, Ethylhexyl Salicylate, Bis-Ethylhexyloxyphenol Methoxyphenyl Triazine, Ethylhexyl Triazone, Butyl Methoxydibenzoylmethane, Titanium Dioxide, Homosalate and Octocrylene (= Preparation A minus Isoamyl p-Methoxycinammate).
Preparation C: 8 UV filters: Disodium Phenyl Dibenzimidazole Tetrasulfonate, Ethylhexyl Salicylate, Bis-Ethylhexyloxyphenol Methoxyphenyl Triazine, Ethylhexyl Triazone, Butyl Methoxydibenzoylmethane, Homosalate, Octocrylene and Isoamyl p-Methoxycinammate (= Preparation A minus Titanium Dioxide).
Preparation D: 8 UV filters: Disodium Phenyl Dibenzimidazole Tetrasulfonate, Ethylhexyl Salicylate, Bis-Ethylhexyloxyphenol Methoxyphenyl Triazine, Butyl Methoxydibenzoylmethane, Titanium Dioxide, Homosalate, Octocrylene and Isoamyl p-Methoxycinammate (= Preparation A minus Ethylhexyl Triazone).
Preparation E: 8 UV filters: Disodium Phenyl Dibenzimidazole Tetrasulfonate, Ethylhexyl Salicylate, Bis-Ethylhexyloxyphenol Methoxyphenyl Triazine, Ethylhexyl Triazone, Titanium Dioxide, Homosalate, Octocrylene and Isoamyl p-Methoxycinammate. (= Preparation A minus Butyl Methoxydibenzoylmethane).
Preparation F: 8 UV filters: Disodium Phenyl Dibenzimidazole Tetrasulfonate, Ethylhexyl Salicylate, Ethylhexyl Triazone, Butyl Methoxydibenzoylmethane, Titanium Dioxide, Homosalate, Octocrylene and Isoamyl p-Methoxycinammate (= Preparation A minus Bis-Ethylhexyloxyphenol Methoxyphenyl Triazine).
Preparation G: 8 UV filters: Disodium Phenyl Dibenzimidazole Tetrasulfonate, Bis-Ethylhexyloxyphenol Methoxyphenyl Triazine, Ethylhexyl Triazone, Butyl Methoxydibenzoylmethane, Titanium Dioxide, Homosalate, Octocrylene and Isoamyl p-Methoxycinammate (= Preparation A minus Ethylhexyl Salicylate).
Preparation H: 8 UV filters: Disodium Phenyl Dibenzimidazole Tetrasulfonate, Ethylhexyl Salicylate, Bis-Ethylhexyloxyphenol Methoxyphenyl Triazine, Ethylhexyl Triazone, Butyl Methoxydibenzoylmethane, Titanium Dioxide, Octocrylene and Isoamyl p-Methoxycinammate (= Preparation A minus Homosalate).
Preparation I: 8 UV filters: Disodium Phenyl Dibenzimidazole Tetrasulfonate, Ethylhexyl Salicylate, Bis-Ethylhexyloxyphenol Methoxyphenyl Triazine, Ethylhexyl Triazone, Butyl Methoxydibenzoylmethane, Titanium Dioxide, Homosalate and Isoamyl p-Methoxycinammate (= Preparation A minus Octocrylene).
Preparation J: 8 UV filters: Ethylhexyl Salicylate, Bis-Ethylhexyloxyphenol Methoxyphenyl Triazine, Ethylhexyl Triazone, Butyl Methoxydibenzoylmethane, Titanium Dioxide, Homosalate, Octocrylene and Isoamyl p-Methoxycinammate. (= Preparation A minus Disodium Phenyl Dibenzimidazole Tetrasulfonate).

The following table shows the measured solar protection factor (SPF) values before and after solar radiation, the UV-A (UVA) protection factor before and after UV irradiation and the % photoactivation in the preceding cases, together with the SPF values that can be indicated (SPF being 20, 30, 50₊ or even 100₊).

**Table 5. Solar protection factor values and UV protection factor values before and after irradiation for preparations A-J.**

| Preparation | Before SPF | After SPF | Before UVA | After UVA | % Photoactivation (SPF) | % Photoactivation (UVA) | SPF |
|---|---|---|---|---|---|---|---|
| A | 32,0 | 61,6 | 27,6 | 48,0 | 92,5 | 73,9 | 50₊ |
| B | 31,1 | 41,1 | 26,9 | 38,8 | 36,5 | 44,2 | 30 |
| C | 26,9 | 37,9 | 23,0 | 32,9 | 40,9 | 43,0 | 30 |
| D | 20,6 | 29,4 | 19,8 | 29,3 | 42,7 | 48,0 | 20 |
| E | 21,3 | 32,5 | 12,3 | 16,8 | 52,6 | 36,6 | 30 |
| F | 24,0 | 37,0 | 19,5 | 29,8 | 54,2 | 54,2 | 30 |
| G | 21,8 | 30,4 | 18,6 | 25,9 | 39,4 | 39,4 | 30 |
| H | 25,5 | 40,7 | 22,4 | 34,9 | 59,6 | 55,8 | 30 |
| I | 23,5 | 36,4 | 21,7 | 35 | 54,9 | 61,3 | 30 |
| J | 121,2 | 92,4 | 71,1 | 54,6 | -23,8 | -23,2 | 100₊ |

Table 5 shows that for the photoactivation to take place, Disodium Phenyl Dibenzimidazole Tetrasulfonate is a mandatory component. Indeed, even though sample J showed the highest SPF, no photoactivation was observed. Thus the Applicant has found that the presence of Disodium Phenyl Dibenzimidazole Tetrasulfonate in the composition is essential for the photoactivation to take place.

Moreover, when reducing the amount of filters from 8-9 to 6, keeping Disodium Phenyl Dibenzimidazole Tetrasulfonate in the selection, the photoactivation in the cosmetic preparation is maintained, as shown in the following table:

**Table 6. Solar protection factor values and UV protection factor values before and after irradiation for preparations K and L.**

| Preparation | Before SPF | After SPF | Before UVA | After UVA | % Photoactivation (SPF) | % Photoactivation (UVA) | SPF |
|---|---|---|---|---|---|---|---|
| K | 49,3 | 80,3 | 40,7 | 53,3 | 62,9 | 31,0 | 50₊ |
| L | 34,8 | 41,0 | 22,0 | 22,6 | 17,8 | 2,7 | 30 |

Therefore, Table 6 shows that reducing the relative amount of UV filters to approximately 50% (from Preparation K to L) did not hamper the photoactivation of the cosmetic preparations. An expected decrease in both the SPF and the amount of photoactivation was also confirmed.

Preparations M to P were designed to better understand the factors involved in the photoactivation phenomenon, taking preparation K as reference.

As described hereinabove, preparation K is a reference preparation according to the present invention. Among the different components included therein, six UV filters are present, together with an amount of water between 30 and 40% by weight.

Preparation M is based on preparation K but without the presence of the antioxidants.

Preparation N is based on preparation K but the content of water has increased up to a value close to 50% w/w while keeping the %w/w of the UV filters as in Preparation M.

Preparation O is based on preparation N but the UV filter of Disodium Phenyl Dibenzimidazole Tetrasulfonate is not present.

Preparation P is based on preparation K but without Ethylhexyl Triazone and a lower amount of Bis-Ethylhexyloxyphenol Methoxyphenyl Triazine.

Photoactivation studies were performed in the preparations M to Q as described hereinabove, and the results obtained are shown in the following table:

**Table 7. Solar protection factor values and UV protection factor values before and after irradiation for preparations K, M, N, O and P.**

| Preparation | Before SPF | After SPF | Before UVA | After UVA | % Photoactivation (SPF) | % Photoactivation (UVA) | SPF |
|---|---|---|---|---|---|---|---|
| K | 49,3 | 80,3 | 40,7 | 53,3 | 62,9 | 31,0 | 50₊ |
| M | 40,8 | 62,8 | 30,5 | 39 | 53,9 | 27,9 | 50₊ |
| N | 22,6 | 36 | 19,5 | 27,5 | 59,3 | 41,0 | 30 |
| O | 41,7 | 39,5 | 23,4 | 22,6 | -5,3 | -3,4 | 30 |
| P | 11,1 | 9,2 | 12,8 | 10,8 | -17,1 | -15,6 | 10 |

The results of Table 7 show that the presence of antioxidants as in preparation K are not relevant to the photoactivity of the cosmetic preparation nor to the solar protection factor. Preparation M shows slightly lower values of photoactivation but keeps the value of SPF of preparation K.

Therefore, this result suggests that the presence of antioxidants in the cosmetic preparation are not essential for the cosmetic preparation to be photoactive and have a convenient SPF.

Preparation N, comprising a higher amount of water while keeping the UV filters of preparation K, show similar photoactivation values but the SPF decreases substantially (from 50₊ to 30).

Therefore, this result suggests that the amount of water in the preparation is critical to obtain a convenient SPF.

Preparation O does not show photoactivation and the SPF value decreases substantially when compared with preparation according to the invention K.

Therefore, this result suggests, as discussed above, that the presence of UV filter Disodium Phenyl Dibenzimidazole Tetrasulfonate is essential for both the photoactivation and the obtention of a convenient SPF.

Preparation P does not show photoactivation and the SPF value dramatically decreases when compared with preparation according to the invention K.

Therefore, this result suggests that the presence of Ethylhexyl Triazone (in combination with Disodium Phenyl Dibenzimidazole Tetrasulfonate) is also essential for the photoactivation to occur and to have acceptable SPF values in a cosmetic preparation when the preparation does not comprise Homosalate, Octocrylene and Isoamyl p-Methoxycynammate. Note that preparation D did photoactivate although the values of SPF were lower than in preparation K. Moreover, in preparation P the amount of Bis-Ethylhexyloxyphenol Methoxyphenyl Triazine was significantly decreased when compared to preparation K, and therefore, this UV filter appears also relevant to the photoactivation and SPF values.

The present disclosure, therefore, shows a phenomenon that has not been previously described (photoactivation of a cosmetic preparation) and the essential ingredients for a composition in a cosmetic preparation to obtain this phenomenon together with a solar protection factor that is convenient to the human skin. The combination of the UV filters Disodium Phenyl Dibenzimidazole Tetrasulfonate, Ethylhexyl Triazone and Bis-Ethylhexyloxyphenol Methoxyphenyl Triazine and a defined percentage of water provides with a composition that is photoactive and has an optimal SPF.

The main advantages of the cosmetic preparation herein disclosed can be summarized as follows: 1) Increased security in the protection against sun radiation, since the cosmetic preparation increases its efficacy when exposed to solar radiation; 2) Possibility to achieve a desired (and high) SPF with a low number and percentage of solar filters; and 3) Cost-efficient and sustainable formulation since lower number and percentage of filters involves higher cost-efficiency, sustainability and protection of the sea and ocean.

## Claims

1. Use of a composition comprising:
a UV filter combination of Disodium 2,2'-(1,4-phenylene)bis(6-sulfo-1H-1,3-benzimidazole-4-sulfonate) (INCI: Disodium Phenyl Dibenzimidazole Tetrasulfonate), 2,2'-[6-(4-Methoxyphenyl)-1,3,5-triazine-2,4-diyl]bis{5-[(2-ethylhexyl)oxy]phenol} (INCI: Bis-Ethylhexyloxyphenol Methoxyphenyl Triazine), 4-[[4,6-bis[[4-(2-ethylhexoxy-oxomethyl)phenyl]amino]-1,3,5-triazin-2-yl]amino]benzoic acid 2-ethylhexyl ester (INCI:
Ethylhexyl Triazone); and
preferably an antioxidant,
to increase the absorbance of a cosmetic preparation.

2. The use according to claim 1, wherein the UV filter combination further comprises at least three compounds selected from the group of the six compounds consisting of 2-ethylhexyl 2-hydroxybenzoate (INCI: Ethylhexyl Salicylate), 3-(4-tert-Butylphenyl)-1-(4-methoxyphenyl)propane-1,3-dione (INCI: Butyl Methoxydibenzoylmethane), Titanium Dioxide, 3,3,5-Trimethylcyclohexyl 2-hydroxybenzoate (INCI: Homosalate), 2-Ethylhexyl 2-cyano-3,3-diphenylprop-2-enoate (INCI: Octocrylene) and 3-Methylbutyl (2E)-3-(4-methoxyphenyl)prop-2-enoate (INCI: Isoamyl p-Methoxycinammate).

3. The use according to claims 1 or 2, wherein the UV filter combination comprises Disodium 2,2'-(1,4-phenylene)bis(6-sulfo-1H-1,3-benzimidazole-4-sulfonate) (INCI: Disodium Phenyl Dibenzimidazole Tetrasulfonate), 2,2'-[6-(4-Methoxyphenyl)-1,3,5-triazine-2,4-diyl]bis{5-[(2-ethylhexyl)oxy]phenol} (INCI: Bis-Ethylhexyloxyphenol Methoxyphenyl Triazine), and 4-[[4,6-bis[[4-(2-ethylhexoxy-oxomethyl)phenyl]amino]-1,3,5-triazin-2-yl]amino]benzoic acid 2-ethylhexyl ester (INCI: Ethylhexyl Triazone), 2-ethylhexyl 2-hydroxybenzoate (INCI: Ethylhexyl Salicylate), 3-(4-tert-Butylphenyl)-1-(4-methoxyphenyl)propane-1,3-dione (INCI: Butyl Methoxydibenzoylmethane) and Titanium Dioxide.

4. The use according to any one of claims 1 to 3, wherein the composition comprises water in an amount of 30% to 40% with respect to the total amount of the preparation.

5. The use according to any one of claims 1 to 4, wherein the composition does not comprise Troxerutin or a fluorescence quencher of disodium phenyl dibenzimidazole tetrasulfonate (INCI: Disodium Phenyl Dibenzimidazole Tetrasulfonate).

6. Cosmetic preparation comprising:
a UV filter combination of
Disodium 2,2'-(1,4-phenylene)bis(6-sulfo-1H-1,3-benzimidazole-4-sulfonate) (INCl: Disodium Phenyl Dibenzimidazole Tetrasulfonate), 2,2'-[6-(4-Methoxyphenyl)-1,3,5-triazine-2,4-diyl]bis{5-[(2-ethylhexyl)oxy]phenol} (INCI: Bis-Ethylhexyloxyphenol Methoxyphenyl Triazine), and 4-[[4,6-bis[[4-(2-ethylhexoxy-oxomethyl)phenyl]amino]-1,3,5-triazin-2-yl]amino]benzoic acid 2-ethylhexyl ester (INCI: Ethylhexyl Triazone), and
at least three compounds selected from the group of the eight compounds consisting of 2-ethylhexyl 2-hydroxybenzoate (INCI: Ethylhexyl Salicylate), 3-(4-tert-Butylphenyl)-1-(4-methoxyphenyl)propane-1,3-dione (INCI: Butyl Methoxydibenzoylmethane), Titanium Dioxide, 3,3,5-Trimethylcyclohexyl 2-hydroxybenzoate (INCI: Homosalate), 2-Ethylhexyl 2-cyano-3,3-diphenylprop-2-enoate (INCI: Octocrylene) and 3-Methylbutyl (2E)-3-(4-methoxyphenyl)prop-2-enoate (INCI: Isoamyl p-Methoxycinammate);
preferably an antioxidant; and
water in an amount of 30% to 40% with respect to the total amount of the preparation.

7. The cosmetic preparation according to claim 6, wherein the UV filter combination comprises Disodium 2,2'-(1,4-phenylene)bis(6-sulfo-1H-1,3-benzimidazole-4-sulfonate) (INCI: Disodium Phenyl Dibenzimidazole Tetrasulfonate), 2,2'-[6-(4-Methoxyphenyl)-1,3,5-triazine-2,4-diyl]bis{5-[(2-ethylhexyl)oxy]phenol} (INCI: Bis-Ethylhexyloxyphenol Methoxyphenyl Triazine), and 4-[[4,6-bis[[4-(2-ethylhexoxy-oxomethyl)phenyl]amino]-1,3,5-triazin-2-yl]amino]benzoic acid 2-ethylhexyl ester (INCI: Ethylhexyl Triazone), 2-ethylhexyl 2-hydroxybenzoate (INCI: Ethylhexyl Salicylate), 3-(4-tert-Butylphenyl)-1-(4-methoxyphenyl)propane-1,3-dione (INCI: Butyl Methoxydibenzoylmethane) and Titanium Dioxide.

8. The cosmetic preparation according to claims 7 or 8, comprising:
a UV filter combination of Disodium 2,2'-(1,4-phenylene)bis(6-sulfo-1H-1,3-benzimidazole-4-sulfonate) (INCI: Disodium Phenyl Dibenzimidazole Tetrasulfonate), of 2-ethylhexyl 2-hydroxybenzoate (INCl: Ethylhexyl Salicylate), 2,2'-[6-(4-Methoxyphenyl)-1,3,5-triazine-2,4-diyl]bis{5-[(2-ethylhexyl)oxy]phenol} (INCI: Bis-ethylhexyloxyphenol Methoxyphenyl Triazine), 4-[[4,6-bis[[4-(2-ethylhexoxy-oxomethyl)phenyl]amino]-1,3,5-triazin-2-yl]amino]benzoic acid 2-ethylhexyl ester (INCI: Ethylhexyl Triazone), 3-(4-tert-Butylphenyl)-1-(4-methoxyphenyl)propane-1,3-dione (INCI: Butyl Methoxydibenzoylmethane), Titanium Dioxide, 3,3,5-Trimethylcyclohexyl 2-hydroxybenzoate (INCI: Homosalate), 2-Ethylhexyl 2-cyano-3,3-diphenylprop-2-enoate (INCI: Octocrylene) and 3-Methylbutyl (2E)-3-(4-methoxyphenyl)prop-2-enoate (INCI: Isoamyl p-Methoxycinammate).

9. The cosmetic preparation according to any one of the previous claims, **characterized in that** the preparation is in the form of an O/W emulsion, a W/O emulsion, a silicone-in-water (Si/W) emulsion, a water-in-silicone (W/Si) emulsion, a stick or a polymeric emulsion.

10. The cosmetic preparation according to any one of the previous claims, wherein the composition does not comprise Troxerutin or a fluorescence quencher of Disodium Phenyl Dibenzimidazole Tetrasulfonate (INCI: Disodium Phenyl Dibenzimidazole Tetrasulfonate).

11. Method for increasing the absorbance of a cosmetic preparation, the cosmetic preparation comprising a composition that comprises
a UV filter combination of Disodium 2,2'-(1,4-phenylene)bis(6-sulfo-1H-1,3-benzimidazole-4-sulfonate) (INCI: Disodium Phenyl Dibenzimidazole Tetrasulfonate), 2,2'-[6-(4-Methoxyphenyl)-1,3,5-triazine-2,4-diyl]bis{5-[(2-ethylhexyl)oxy]phenol} (INCI: Bis-Ethylhexyloxyphenol Methoxyphenyl Triazine), and 4-[[4,6-bis[[4-(2-ethylhexoxy-oxomethyl)phenyl]amino]-1,3,5-triazin-2-yl]amino]benzoic acid 2-ethylhexyl ester (INCI:
Ethylhexyl Triazone), and
preferably an antioxidant;
the method comprising the step of irradiating the cosmetic preparation with UV light.

12. The method according to claim 11, wherein the UV filter combination further comprises at least three compounds selected from the group of the six compounds consisting of 2-ethylhexyl 2-hydroxybenzoate (INCI: Ethylhexyl Salicylate), 3-(4-tert-Butylphenyl)-1-(4-methoxyphenyl)propane-1,3-dione (INCI: Butyl Methoxydibenzoylmethane), Titanium Dioxide, 3,3,5-Trimethylcyclohexyl 2-hydroxybenzoate (INCI: Homosalate), 2-Ethylhexyl 2-cyano-3,3-diphenylprop-2-enoate (INCI: Octocrylene) and 3-Methylbutyl (2E)-3-(4-methoxyphenyl)prop-2-enoate (INCI: Isoamyl p-Methoxycinammate).

13. The method according to claims 11 or 12, wherein the UV filter combination comprises Disodium 2,2'-(1,4-phenylene)bis(6-sulfo-1H-1,3-benzimidazole-4-sulfonate) (INCl: Disodium Phenyl Dibenzimidazole Tetrasulfonate), 2,2'-[6-(4-Methoxyphenyl)-1,3,5-triazine-2,4-diyl]bis{5-[(2-ethylhexyl)oxy]phenol} (INCI: Bis-Ethylhexyloxyphenol Methoxyphenyl Triazine), and 4-[[4,6-bis[[4-(2-ethylhexoxy-oxomethyl)phenyl]amino]-1,3,5-triazin-2-yl]amino]benzoic acid 2-ethylhexyl ester (INCI: Ethylhexyl Triazone), 2-ethylhexyl 2-hydroxybenzoate (INCI: Ethylhexyl Salicylate), 3-(4-tert-Butylphenyl)-1-(4-methoxyphenyl)propane-1,3-dione (INCI: Butyl Methoxydibenzoylmethane) and Titanium Dioxide.

14. The method according to any one of claims 11 to 13, wherein the UV light is UV-A and/or UV-B light.

15. Method for increasing the absorbance of a cosmetic preparation comprising a composition according to any one of claims 6 to 10, the method comprising the step of irradiating the cosmetic preparation with UV light.
